# EUROPEAN PATENT APPLICATION

(11) **EP 2 873 676 A1**
(43) Date of publication of application: **20.05.2015**
(21) Application number: 12875413.2
(22) Date of filing: 25.05.2012
(51) Int. Cl.: C07K 7/06, C07K 1/13, A61K 51/08, A61K 101/02, A61K 103/00, A61K 103/10, A61P 35/00

(54) **SMALL MOLECULAR PEPTIDE AND USE THEREOF**

(30) Priority: 25.04.2012 CN 201210123601; 25.04.2012 CN 201210123590
(71) Applicant: Zhujiang Hospital, Southern Medical University, Guangzhou, Guangdong 510282 (CN)
(72) Inventor: GUO, Linlang, Guangzhou Guangdong 510282 (CN); LI, Guiping, Guangzhou Guangdong 510282 (CN); CHEN, Zhenzhu, Guangzhou Guangdong 510282 (CN); LIU, Yajie, Guangzhou Guangdong 510282 (CN)
(74) Representative: Hryszkiewicz, Danuta
(86) International application number: PCT/CN2012/076102
(87) International publication number: WO 2013/159422

(57) **Abstract**

Provided in the present invention is a small molecular peptide capable of binding specifically with lung cancer cells; the peptide is a molecule with 8 amino acids containing the structure of XGXG, wherein G is L-glycine and X is one of 20 amino acids. Also provided in the present invention are a small molecular probe obtained by labeling the above-mentioned small molecular peptide with isotope 99mTc, a small molecular radio-therapeutic agent targeting lung cancer obtained after labeling the above-mentioned small molecular peptide with isotope ¹³¹I and preparation methods for the above-mentioned probe and therapeutic agent.

## Description

### FIELD OF THE INVENTION

The invention relates to the field of biomedical technology, and more particularly to a small peptide for preparing a small peptide probe and targeted radiotherapeutic agent specifically for imaging diagnosis and treatment of lung cancer.

### BACKGROUND OF THE INVENTION

Lung cancer is the most common cancers and is the leading cause of cancer death especially in city of China. The major cause of high mortality rate of lung cancer is due to over 80% of patients in lately stage and missing of optimal opportunity for treatment. Meanwhile, the five-year survival rate for patients with stage I lung cancer is up to 80 percent. Therefore, early diagnosis will help to improve survival and reduce mortality of lung cancer patients. Globally, imaging technology remains the most effective methods for lung cancer detection. Modern equipment and advanced technique have significantly increased early diagnosis rate of lung cancer. However, the sensitivity and specificity of these current scanning modalities such as CT, PET or MIR need to be further improved. Molecular imaging has provided a very important platform to set up the highly sensitive and specific method for detection of lung cancer. Molecular probe is a crucial factor for molecular imaging technique, as well as one of the prerequisite for molecular imaging research. According to the imaging technology, the widely used molecular probes are classified as follows: (1) Nuclear imaging probes. Isotopically-labeled integrin αᵥβ₃ and VEGF probes have demonstrated very satisfying imaging of tumor angiogenesis in animal models. (2) MRI imaging probes. MRI combined with Gd-DTPA labeled monocolonal antibody integrin αᵥβ₃ or small peptide RGD can dramatically improve imaging sensitivity of MRI in rabbit or nude mouse models. (3) Optical imaging probes. Due to the limited penetration of the optical imaging technique, optical imaging probes is only used for research in small animal models.

Comparing with large molecular antibody probes, small peptide probes have several advantages such as small size, implying good tissue diffusion and target accessibility, no antigenicity, easy synthesis, low production cost, adequate radiolabeling and so on. Therefore, the small peptide probes have demonstrated much better prospects for clinical application than larger molecular probes. The small peptide-based probes for cancer imaging in clinical studies are presently as follows: (1) the radioactive somatostatin-based probes for diagnosis of gastrointestinal and bronchial neuroendocrine cancer, pituitary adenoma, paraganglioma and so on; (2) CCK (cholecystokinin) probes for diagnosis of medullary thyroid cancer diagnosis; (3) bombesin probes for diagnosis of breast cancer and prostate cancer; (4) RGD probes for vascular imaging in a variety of solid tumors. Thus far, there have been no successful research reports of lung cancer imaging using specific molecular probes at home and abroad.

In addition, application for molecularly targeted agents in lung cancer has been showing a future prospect and promising trend. These targeted anticancer agents promise more efficient and less toxic side effects for the advanced lung cancer patients. However, the current clinically used targeting anticancer drugs, including Iressa, Tarceva, ENDOSTAR, EGFR receptor inhibitors or VEGF receptor inhibitors, are required long-term use and effective for a small percentage of patients with an EGFR mutation. High medical costs usually make cancer patients give up treatment. At present, Iressa used clinically in China, has been strictly limited its clinical application in the United States by FDA because it failed to prolong survival in lung cancer clinical trials.

Besides the target anticancer drugs, radioimmunotherapy (RIT) is another promising approach to targeting cancer therapy. Application of yttrium-90 (⁹⁰Y)-ibritumomabtiuxetan (Zevalin) and iodine-131 (¹³¹I)-tositumomab (Bexxar) has achieved remarkable success in the treatment of lymphoma with an annual turnover of one billion dollars. RIT has become another important targeted therapeutic strategy for cancer treatment except for chemotherapy.

¹³¹I-chTNT is the world's first radioimmunotherapy agent for the treatment of solid tumors and the third RIA drug followed by the global current status of zevalin and bexxar. In January 2007, ¹³¹I-chTNT has been officially approved in China for the treatment of solid tumors including lung cancer, hepatocellular carcinoma and brain tumor. Clinical trials showed the ORR (objective response rate) was 33% in patients with advanced lung cancer. However, ¹³¹I-chTNT has obvious defects including its potential allergen because of derived components in recombinant chimeric TNT antibody, a non-specific aggregation easily recognized and phagocytosed by reticuloendothelial system, the poor penetration in tumor tissue because of its large molecular weight. All these difficult issues will affect its clinic efficacy.

### SUMMARY OF THE INVENTION

One object of this invention is to provide a small peptide specifically binding to lung cancer cells.

Another object of the invention is to provide a specific small peptide probe for imaging diagnosis of lung cancer and its preparation method.

Another object of the invention is to provide a small molecular targeted radiotherapeutic agent for lung cancer and its preparation method.

The technical schemes of the invention are as follow:

A small peptide comprises 8 amino acids with a sequence of XGXG, where: "G" represents L- Glycine (Gly), "X" represents any one of 20 amino acids.

The amino acid sequence of the peptide stated above is cNGEGQQc (SEQ ID NO.1), where: "c" represents d-cysteine (Cys), "N" represents L-Asparagine (Asn), "G" represents L-Glycine (Gly), "E" represents L-Glutamic acid (Glu), "Q" represents L-Glutamine (Gln).

A small peptide probe is obtained by labeling a peptide with a radiotherapeutic isotope. The peptide comprises 8 amino acids with the sequence of XGXG, where: "G" represents L- Glycine (Gly), "X" represents any one of 20 amino acids.

The small peptide probe described above comprises 8 amino acids with the sequence of cNGEGQQc (SEQ ID NO.1), wherein: "c" represents d-cysteine (Cys), "N" represents L-Asparagine (Asn), "G" represents L-Glycine (Gly), "E" represents L-Glutamic acid (Glu), "Q" represents L-Glutamine (Gln).

The radiotherapeutic isotope is ^{99m}Tc, ¹¹¹In, ¹⁸F-FDG, ⁶⁸Ga, or ⁶⁴Cu.

A method for producing the small peptide probe comprises the following steps:

(1) conjugating the peptide to a chelator of S-acetyl-mercaptoacetyltriglycine (MAG₃) to produce a small peptide-MAG₃ complex; and

(2) labeling the peptide-MAG₃ complex by a radiotherapeutic isotope to generate the small peptide probe.

The peptide comprises 8 amino acids with the sequence of XGXG, where: "G" represents L- Glycine (Gly), "X" represents any one of 20 amino acids.

The radiotherapeutic isotope is ^{99m}Tc.

The small peptide probe described above comprises 8 amino acids with the sequence of cNGEGQQc (SEQ ID NO.1), wherein: "c" represents d-cysteine (Cys), "N" represents L-Asparagine (Asn), "G" represents L-Glycine (Gly), "E" represents L-Glutamic acid (Glu), "Q" represents L-Glutamine (Gln).

The small peptide probe described above is used for producing a molecular imaging diagnostic agent of lung cancer.

A small molecular radiotherapeutic agent for lung cancer is obtained by labeling the peptide with a radiotherapeutic isotope. The peptide comprises 8 amino acids with the sequence of XGXG, where: "G" represents L- Glycine (Gly), "X" represents any one of 20 amino acids.

The peptide described above comprises 8 amino acids with the sequence of cNGEGQQc (SEQ ID NO.1), wherein: "c" represents d-cysteine (Cys), "N" represents L-Asparagine (Asn), "G" represents L-Glycine (Gly), "E" represents L-Glutamic acid (Glu), "Q" represents L-Glutamine (Gln).

The radiotherapeutic isotope is ¹³¹I, ¹⁸⁸Re, or ⁹⁰Y.

A method for producing the small molecular targeted radiotherapeutic agent for lung cancer comprises the following formula:
(1) coupling a N-terminal of a peptide to tyrosine; and
(2) dissolving the peptide coupling with tyrosine on its N-terminal, adding ¹³¹I and chloramine-T respectively, mixing a resulting mixture in a shaker for 2 min, and adding sodium thiosulfate for stopping the reaction.

The peptide comprises 8 amino acids with the sequence of XGXG, where: "G" represents L- Glycine (Gly), "X" represents any one of 20 amino acids.

The radiotherapeutic isotope is ¹³¹I.

The peptide comprises 8 amino acids with the sequence of cNGEGQQc (SEQ ID NO.1), wherein: "c" represents d-cysteine (Cys), "N" represents L-Asparagine (Asn), "G" represents L-Glycine (Gly), "E" represents L-Glutamic acid (Glu), "Q" represents L-Glutamine (Gln).

Specifically, step (1) is detailed as follows: mixing the small peptide cNGEGQQc and tyrosine, adding a condensing agent EDC-HCl (1-ethyl-(3-dimethylaminopropyl) carbodiimide hydrochloride amine), allowing a resulting mixture to reaction at room temperature for 2 hours so that the N-terminal of the peptide is coupled to C-terminal of tyrosine by condensation reaction.

Specifically, step (2) is detailed as follows: diluting peptide-Tyr complex in PBS buffer (0.5 M, pH=7.4); after the peptide is completely dissolved in the buffer, adding ¹³¹I (37 MBq/20 µl) and chloramine-T (final concentration 0.9 µg/µl). The mixture is mixed in a shaker for 2 min and then adding 45 µl of sodium thiosulfate (4 µg/µl) for stopping the reaction.

The beneficial effects of the invention are as follows
(1) This invention relates to a peptide specifically binding to lung cancer cells. The peptide provides a novel method for specific diagnosis and treatment of lung cancer.
(2) This invention provides a small peptide probe preparing by ^{99m}Tc labeled peptide. The small peptide probe is able to image cancer cells in vivo and can be applied to molecular imaging diagnosis and differential diagnosis for lung cancer. It has presented important clinical significance in promoting early diagnosis rate of lung cancer because of good sensitivity and specificity.
(3) This invention provides a small molecular targeted radiotherapeutic agent for lung cancer preparing by therapeutic radionuclide ¹³¹I labeled peptide. The radiotherapeutic agent is characterized by good stability and biological distribution. In mice bearing lung cancer xenografts, radiotherapeutic agent can significantly inhibit tumor growth *in vivo* and has no obvious toxicity to major organs including liver, kidneys, lungs and heart. The radiotherapeutic agent can be applied to targeted therapy for non-small cell lung cancer (squamous cell carcinoma and adenocarcinoma). It has presented important clinical significance in molecular targeted therapies for lung cancer.

### BRIEF DESCRIPTION OF THE DRAWINGS

FIG. 1 shows mass spectrometry (MS) analysis of cNGEGQQc-MAG3;
FIG. 2 shows high performance liquid chromatography (HPLC) analysis of cNGEGQQc-MAG3;
FIG. 3 shows stability testing of molecular imaging probe ^{99m}Tc -cNGEGQQc by paper chromatography (A. acetone system; B. ammonia/ethanol/water mixture system);
FIG. 4 shows biodistribution of ^{99m}Tc-cNGEGQQc in mice;
FIG. 5 shows biodistribution of ^{99m}Tc-cNGEGQQc in rabbits by SPECT scanning for one minute (1 frame/second);
FIG. 6 shows biodistribution of ^{99m}Tc-cNGEGQQc in rabbits by SPECT scanning for 5 minutes (1 frame/second);
FIG. 7 shows biodistribution of ^{99m}Tc-cNGEGQQc in rabbits by SPECT scanning for 30 minutes (1 frame/5 minutes);
FIG. 8 shows the time-radioactivity curves of heart, liver, spleen, kidney and bladder were measured using the region-of-interest (ROI)-based analysis by injection of ^{99m}Tc-cNGEGQQc (1. the time-radioactivity curve from ROI analysis after dynamic recording in major organs of normal rats; 2. the time-radioactivity curve of heart; 3. the time-radioactivity curve of spleen; 4 the time-radioactivity curve of liver; 5. the time-radioactivity curve of kidney; 6. the time-radioactivity curve of bladder);
FIG. 9 shows the anterior and rear images were acquired by SPECT scanning after 1.5 h intravenous injection of ^{99m}Tc-cNGEGQQc;
FIG. 10 shows SPECT imaging of mice bearing L78 tumors were obtained after intravenous injection of ^{99m}Tc-cNGEGQQc at different time (arrows show tracer uptake in the tumors);
FIG. 11 shows SPECT imaging of mice bearing H1975 tumors were obtained after intravenous injection of ^{99m}Tc-cNGEGQQc (arrows show tracer uptake in the tumors). (A, after 2h injection of ^{99m}Tc-cNGEGQQc; B, after 2h injection of ^{99m}Tc-cNAQAEQ) (arrows show tracer uptake in the tumors);
FIG. 12 shows mass spectrometry (MS) analysis of cNGEGQQc-Tyr;
FIG. 13 shows the radiochemical purity (RCP) of ¹³¹1-cNGEGQQc was analyzed by paper chromatography;
FIG. 14 shows radiochromatograms of ¹³¹1-cNGEGQQc was analyzed by high performance liquid chromatography (HPLC);
FIG. 15 shows the time-radioactivity curves of heart, liver, spleen, kidney and bladder were measured using the region-of-interest (ROI)-based analysis by injection of ¹³¹1-cNGEGQQc (1. the time-radioactivity curve from ROI analysis after dynamic recording in major organs of normal rats; 2. the time-radioactivity curve of heart; 3. the time-radioactivity curve of spleen; 4 the time-radioactivity curve of liver; 5. the time-radioactivity curve of kidney; 6. the time-radioactivity curve of bladder);
FIG. 16 shows the anterior and rear images were acquired by SPECT scanning after intravenous injection of ¹³¹1-cNGEGQQc at different time (1-2, after 30 mins injection of ¹³¹1-cNGEGQQc; 3-4, after 1 h injection of ¹³¹1 -cNGEGQQc; 5-6, after 3.5 h injection of ¹³¹1-cNGEGQQc);
FIG. 17 shows growth curve of H1975 cells in nude mice; and
FIG. 18 shows growth curve of L78 cells in nude mice.

### DETAILED DESCRIPTION OF THE EMBODIMENTS

The following examples are provided in order to demonstrate and further illustrate the invention in detail. However, the examples should not be construed as limiting in any manner.

### Example 1

### 1. Synthesis of small peptide cNGEGQQc (SEQ IN NO.1) using solid-phase peptide synthesis

(1) One gram of the resin beads with NH₂ functional groups was weighted and washed with dimethylformamide (DMF) for three times. Beads were then soaked in DMF to be fully swollen.
(2) After adding to d-cysteine and N, N'-diisopropyl carbodiimide, the reaction time was 2 hours at room temperature. Next, beads were washed with DMF for five times. A solution of 20% (v/v) piperidine was added to the beads for 5 min at room temperature, followed by adding to DMF for 15 min to allow the Fmoc deprotection.
(3) According to the procedure stated above in step 2, L-Asparagine, L-Glycine, L-Glutamic acid, L-Glycine, L-Glutamine, L-Glutamine and d-cysteine were coupled to the beads in order.
(4) When the last amino acid in the sequence was successfully coupled, the beads were washed with 25% (v/v) trifluoroacetic acid once and distilled water three times.
(5) Peptides were cleaved from the beads with anhydrous hydrogen fluoride. All side-chain protecting groups were also removed simultaneously. A disulfide bond in peptides was formed between two cysteines by oxidation with iodine in 30% (v/v) acetic acid. The cleaved peptides were purified using gel filtration on Sephadex G-15 column, followed by high-performance liquid chromatographic purification (HPLC).The purified peptides showed a single major peak by RP-HPLC analysis.

### 2. Preparation of small peptide probe ^{99m}Tc-cNGEGQQc

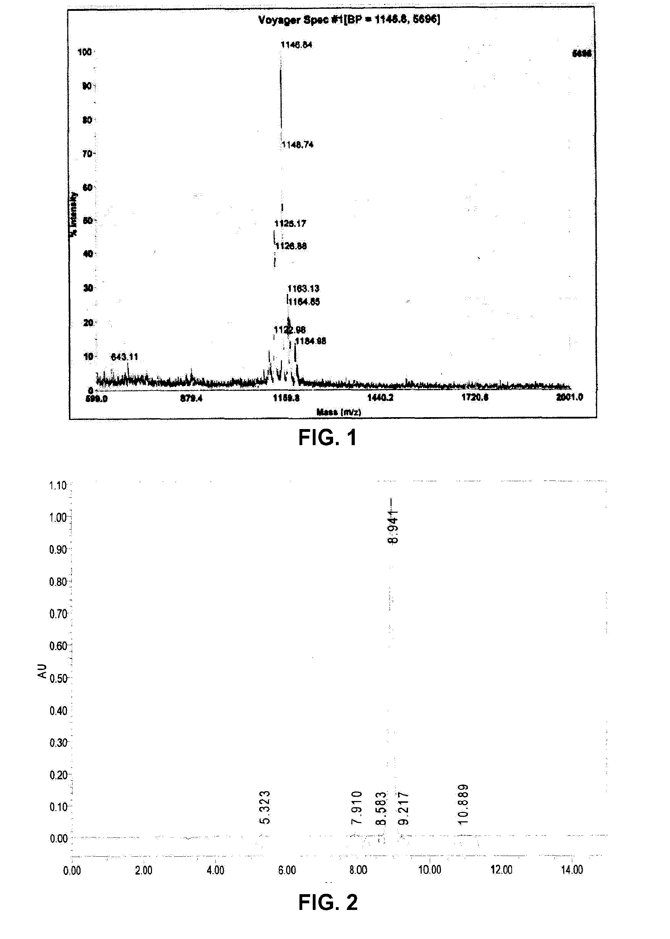

(1) The peptide was conjugated to a chelator of S-acetyl-mercaptoacetyltriglycine (MAG₃) to produce peptide cNGEGQQc-MAG₃ complex. The theoretical molecular weight of the peptide is 1125.05Da, while accurate molecular weight of the purified peptides was determined to be 1146.84Da by mass spectrometry (Fig 1). The purity of peptides was monitored as 96% by HPLC (Fig 2). For conjugation of the peptide-MAG₃ complex, the procedure was performed according to the following manner. i) Synthesis of SATA. S-acetyl mercaptoacetic acid was synthesized from mercaptoacetic acid and acetic anhydride with 1:1.1 molar ratios at room temperature for 4 days. The high purity complex was obtained under reduced pressure (115-125°C, 2-3 mmHg). Both S-acetyl mercaptoacetic acid (75 mmol) and NHS (75 mmol) were dissolved in 150 ml of dioxane, followed by adding to dicyclohexylcarbodiimide (DCC) (75 mmol). The reaction mixture was stirred at 4°C for 16 h. The precipitated dicyclohexylurea was removed by filtration and the solvent evaporated to dryness under vacuum. The residue was crystallized twice with isopropanol to produce STAT. ii) Synthesis of small peptide cNGEGQQc-MAG₃ complex. Peptide cNGEGQQc was prepared by conventional solid-phase synthesis according to the method stated above. During the synthesis, three of glycines were coupled to the N-terminus of peptide. When the last glycine was coupled and deprotected, one-fold molar excess of SATA was added into peptide solution with 7-fold molar excess of 3mDIEA (diisopropylethylamine) and 3-fold molar excess of HBTU (O-Benzotriazole-N,N,N',N'-tetramethyl-uronium- hexafluoro-phosphate) and 3-fold molar excess of HOBT (1-hydroxybenzotriazole). The mixture was incubated at room temperature for 60 min. The reaction was done when Ninhydrin reaction showed in yellow colouration. After completing synthesis, the peptide resin was washed with NMP (N-methylpyrrolidone) and DCM (dichloromethane) alternately for six times, and then removed from the column and dried in vacuo. The peptides were cleaved from the resins by treatment with mixture solution (Iml of ethanedithiol, 1 ml of thioanisole, 0.5g of phenol, 0.4ml of H₂O and 0.1 ml of triisopropylsilyl) for 7h. The crude complex was precipitated with diethyl ether four times and separated by centrifugation and freeze dried. Purified cNGEGQQc-MAG₃ complex was characterized by analytical HPLC. Conjugation of MAG3 using this protocol was done directly during the synthesis of cNGEGQQc.
(2) Indirect labeling method was performed to label cNGEGQQc-MAG₃ complex with ^{99m}Tc according to the reference (Winnard P, et al. Nucl Med Biol, 1997; 24:425-432) with minor improvements. The conjugated peptides cNGEGQQc-MAG₃ (20 µg) were labeled with fresh ^{99m}TcO4⁻ solution (37 MBq) using labeling buffer (0.25 mol/L of Sodium Bicarbonate, 0.125 mol/L of ammonium acetate and 0.18 mol/L of ammonium hydroxide). The reaction solution was thoroughly shaken and incubated in water bath.

We chose the 5 important factors in the 4 different conditions for labeling peptide with ^{99m}Tc: concentration of stannous tartrate at 0.25/1/5/10µg/µl, pH value at 2/4/7.6/10.0, reaction temperature at 25/37/75/100°C, reaction time for 10/20/30/60 min (Reaction was sealed using nitrogen gas), final concentration of potassium sodium tartrate turn chelator at 0.5/1/3.5/10µg/µl. (Table 1). The orthogonal design was carried out to optimize the experimental conditions for labeling (Table 2). The optimal labeling conditions were analyzed by orthogonal design. The design for factors and the levels is shown in Table 1. The experimental results of orthogonal design are shown in Table 2.

**Table 1 Important factors for labeling peptide**

| Level | Temperature (°C) | | Time (min) | pH | Potassium sodium tartrate (µg/µL) | Stannous tartrate (µg/µL) |
|---|---|---|---|---|---|---|
| 1 | 25 | 10 | | 2.0 | 0.5 | 0.25 |
| 2 | 37 | 20 | | 4.0 | 1 | 1 |
| 3 | 75 | 30 | | 7.6 | 3.5 | 5 |
| 4 | 100 | 60 | | 10.0 | 10 | 10 |

**Table 2 Orthogonal design of four factors for labeling peptide**

| No | Temperature (°C) | Time (min) | pH | Potassium sodium tartrate (µg/µL) | Stannous tartrate (µg/µL) | Labeling (%) |
|---|---|---|---|---|---|---|
| 1 | 25 | 10 | 2.0 | 0.5 | 0.25 | 7.50% |
| 2 | 25 | 20 | 4.0 | 1 | 1 | 85.60% |
| 3 | 25 | 30 | 7.6 | 3.5 | 5 | 98.60% |
| 4 | 25 | 60 | 10.0 | 10 | 10 | 89.50% |
| 5 | 37 | 10 | 2.0 | 0.5 | 0.25 | 85.20% |
| 6 | 37 | 20 | 4.0 | 1 | 1 | 89.90% |
| 7 | 37 | 30 | 7.6 | 3.5 | 5 | 98.30% |
| 8 | 37 | 60 | 10.0 | 10 | 10 | 91.50% |
| 9 | 75 | 10 | 2.0 | 0.5 | 0.25 | 85.40% |
| 10 | 75 | 20 | 4.0 | 1 | 1 | 91.50% |
| 11 | 75 | 30 | 7.6 | 3.5 | 5 | 94.80% |
| 12 | 75 | 60 | 10.0 | 10 | 10 | 97.70% |
| 13 | 100 | 10 | 2.0 | 0.5 | 0.25 | 97.60% |
| 14 | 100 | 20 | 4.0 | 1 | 1 | 89.10% |
| 15 | 100 | 30 | 7.6 | 3.5 | 5 | 93.50% |
| 16 | 100 | 60 | 10.0 | 10 | 10 | 10.50% |

The optimal labeling conditions by orthogonal design were as follows: final concentration of potassium sodium tartrate as the chelator is 3.5 µg/µl, 5 µg/µl of stannous tartrate, and reaction conditions are at pH 7.6 and 25°C for 30 min.

### 3. Confirmation of labeling efficiency and in vitro stability of small peptide probe ^{99m}Tc-cNGEGQQc

Labeling efficiency of molecular probe was determined by paper chromatography. The details were as follow: a drop of the labeled small peptide was placed on one side of the paper, then dipped into a mixture liquid with ethanol: ammonia: water (2: 1: 5) (system 1) and acetone (system 2). The separation occurs as the liquid moves along the paper. Take the paper out and dry it when the liquid moves to the other side of paper. Paper was cut into ten equal pieces and put into the tube separately. The radioactivity of each pieces of paper was measured by γ radioactivity counter. The percentage of radioactivity and labeling rate for each peak was calculated according to the radioactivity counts of two peaks. Radioactivity percentage = (radioactivity count / total radioactivity count) X 100%. Labeling rate = radioactive percentage of ^{99m}Tc-labeled peptides in system 1-radioactivity percentage of ^{99m}Tc in system 2). The peptides were labeled using optimal labeling conditions and measured by paper chromatography. The labeling rate of peptide using ^{99m}Tc ranged 84%-95%. The labeling method was proved to be good in reproducibility.

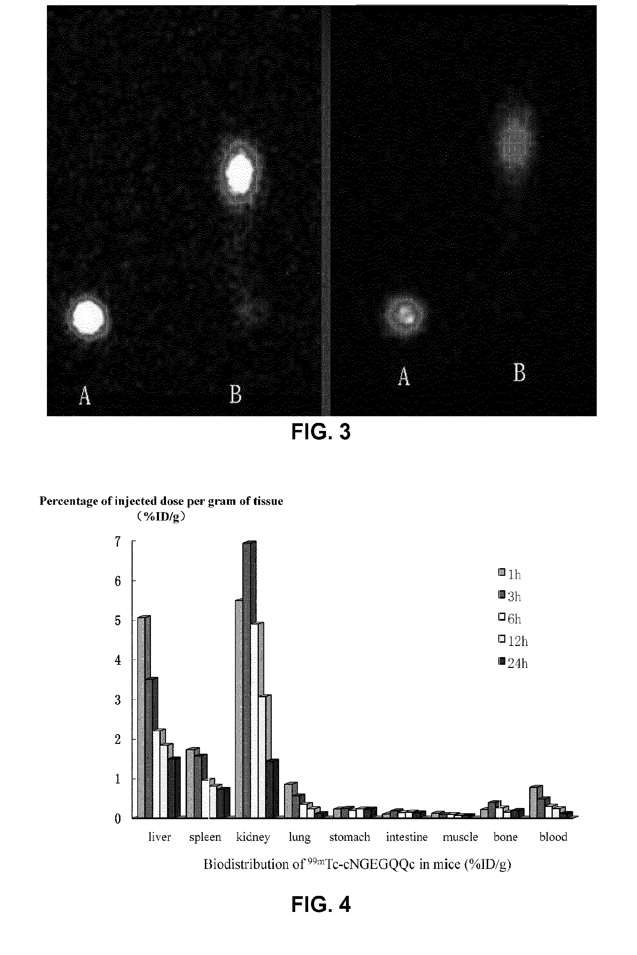

To evaluate the stability of molecular probe in vitro, radiochemical purity was measured using paper chromatography. The formula for radiochemical purity is radioactive percentage of ^{99m}Tc-labeled peptides (system 1)-radioactivity percentage of ^{99m}Tc (system 2). After purification with HPLC, the ^{99m}Tc cNGEGQQc-MAG₃ was placed at room temperature for 24h. The radiochemical purity was 95% at 0 h and 91 % at 24h respectively. To further estimate the stability of small peptide probe in simulation environment in vivo, the probe was placed in fresh human serum at 37°C for 24h. After 24 h incubation, the radiochemical purity was 95% at 0 h and 85% at 24h respectively. These data suggests that the small peptide probe is very stable in serum (Figure 3).

### 4. Analysis of biodistribution of small peptide probe ^{99m}Tc-cNGEGQQc in vivo

(1) Fifteen normal Kunming male mice (4-6 weeks old, weight 19-21 g) were injected with 0.1 ml (2.96MBq) of the small peptide probe ^{99m}Tc-cNGEGQQc via a tail vein respectively. At 1, 3, 6, 12, and 24h, three animals were anesthetized and sacrificed by cervical dislocation. Whole blood was collected and organs of interest were removed and weighed. The amount of radioactivity in blood and each organ was determined and calculate the percentage of the injected dose per gram of tissue (%ID/g).SPECT (Millennium VG; GE Healthcare) was equipped with a low-energy, high resolution collimator. Images were collected using energy peak centered at 140KeV, an energy window of 20% and a 128X128 matrix at a magnification of 1.0.

SPECT imaging showed that the radioactivity of kidney and liver was significantly higher than that of other organs in healthy mice. The uptake of small peptide probe was more and clearance took longer in kidney than that in liver. These results showed that the small peptide probe mainly excreted by kidneys. During the observation period, the radioactivity of various organs gradually decreased, while the radioactivity of gastrointestinal was relatively stable. It indicated that the stability of small peptide probe was excellent stable and no free ^{99m}Tc release in vivo. Only little uptake of the small peptide probe was observed in muscles. The biodistribution of the small peptide probe in mice was shown in Table 3 and Figure 4. Therefore, it will be excellent for imaging of lung cancer patients because of little uptake in lungs, muscles and bones and lower background contrast to tumors.

**Table 3 Biodistribution of ^{99m}Tc-cNGEGQQc in mice (%ID/g)**

| Tissues | 1 h | 3 h | 6 h | 12 h | 24 h |
|---|---|---|---|---|---|
| liver | 5.0643±1.3116 | 3.5032±01.4641 | 2.2112±2.8001 | 1.8420±0.5133 | 1.4979±0.0196 |
| spleen | 1.7332±0.1762 | 1.5724±1.0061 | 0.9598±1.1390 | 0.8098±0.0330 | 0.7272±1.1205 |
| kidney | 5.4965±1.1285 | 6.9413±2.12189 | 4.9006±0.7596 | 3.0748±0.3810 | 1.4445±0.2331 |
| lung | 0.8558±0.1760 | 0.5627±0.4336 | 0.3496±0.0457 | 0.2337±0.1501 | 0.1148±0.0137 |
| stomach | 0.2304±0.0708 | 0.2442±0.0857 | 0.1965±0.1023 | 0.2326±0.0691 | 0.2266±0.0162 |
| intestine | 0.1059±0.0296 | 0.1817±0.0737 | 0.1427±0.0550 | 0.1524±0.0150 | 0.1367±0.0256 |
| muscle | 0.1261±0.0818 | 0.0920±0.01511 | 0.0950±0.0257 | 0.0691±0.0185 | 0.0702±0.0007 |
| bone | 0.2165±0.1083 | 0.3969±0.1026 | 0.2590±0.1329 | 0.1524±0.0315 | 0.1886±0.0723 |
| blood | 0.7801±0.3564 | 0.4845±0.1010 | 0.2987±0.1194 | 0.2387±0.0666 | 0.1205±0.0270 |

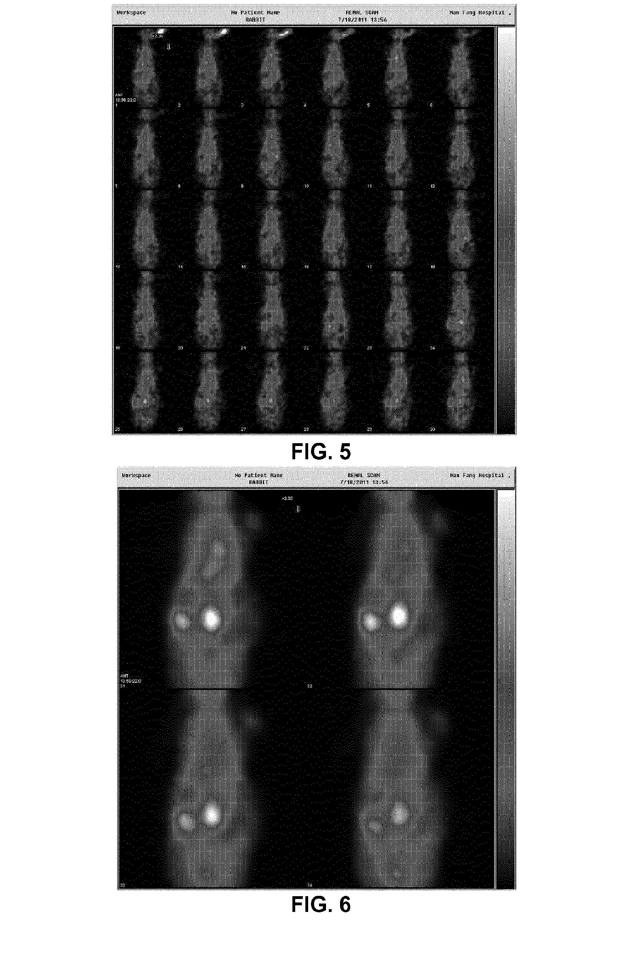

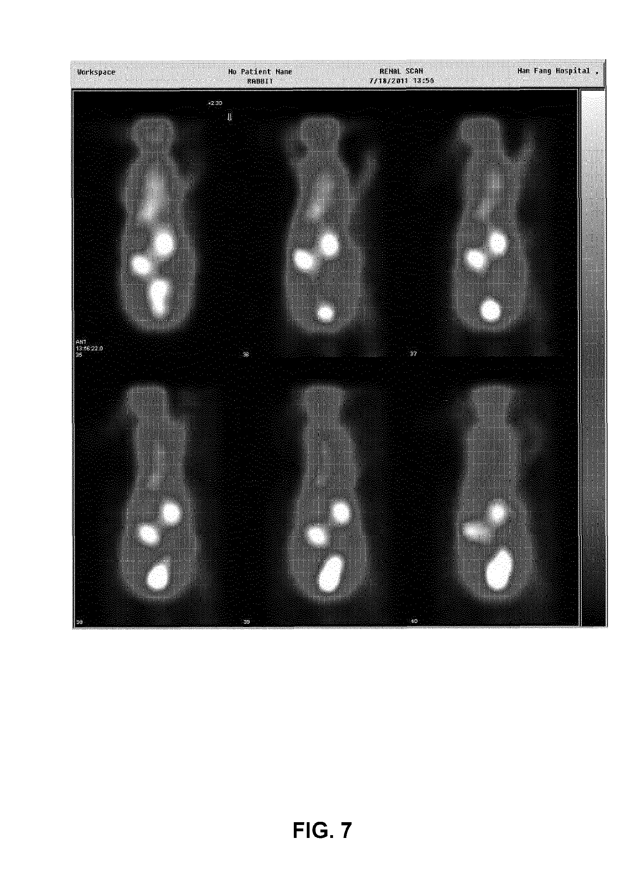

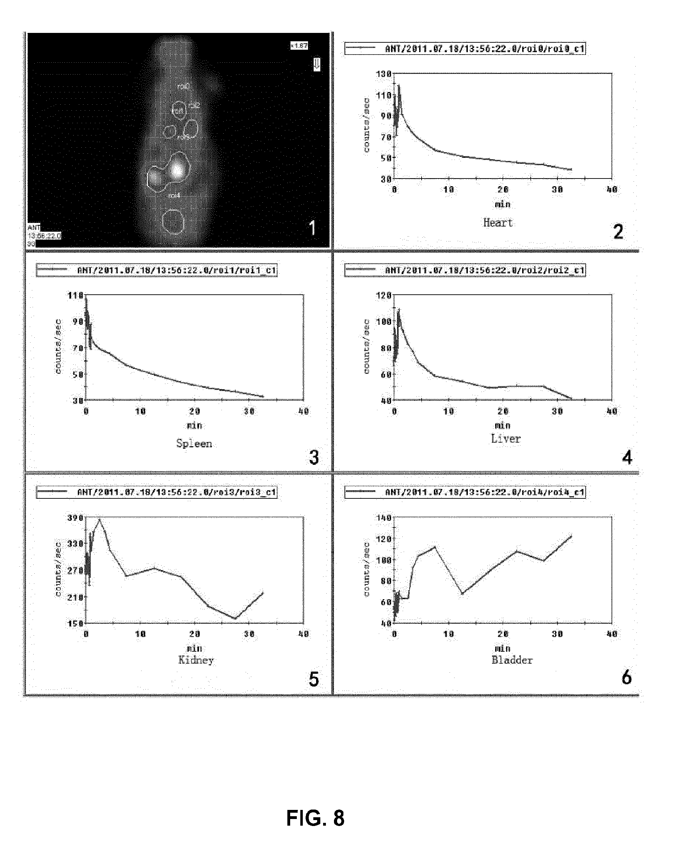

(2) Two of healthy Japanese male white rabbits were fixed in supine position on a wooden experimental stage. SPECT collimator was set on the rabbit thoracic and abdominal to ensure that the whole body of rabbit was within vision field of SPECT imaging. The injectate of small peptide probe ^{99m}Tc-cNGEGQQc diluted with saline water (0.5 ml/74 MBq/per rabbit) was administered through the ear vein injection. Images were acquired immediately and for the next 60 min at a rate of 1 frame/min after injection of molecular probe, followed a rate of 1 frame/2 min at 90 min, 120 min, 180 min and 240min. The distribution of molecular probe in animal organs was observed at different time post injection. The posterior dynamic images were analyzed by ROI. The time-radioactivity curves of main organs including precordia, liver, spleen, kidney and bladder were obtained respectively by ROI analysis (Figure 5-9).

### 5. The diagnostic value of small peptide probe ^{99m}Tc-cNGEGQQc in mice bearing human lung cancer

(1) Animal models of human tumors. Two human lung cancer including NCl-H1975(adenocarcinoma)and L78 (squmous carcinoma) and three other cells including MCF7 (breast carcinoma), HT-29 (colon carcinoma) and HepG2 (hepatocelluar carcinoma) were used in the study. These cells were cultured in conventional methods. When cells grew at logarithmic phase, cells were then harvested with trypsin and centrifuged to remove trypsin digestion solution. After washing with PBS twice, the cells were re-suspended in free serum culture medium at a concentration of 5X10⁶ cells / ml. The suspended cells (0.2 ml) were inoculated into the back of nude mice to establish the cancer models, respectively. There are four nude mice in each group of cancer model. Tumor growth and general states such as mental, diet and weight were monitored periodically. When tumors reached approximately 1 cm in mean diameter, the tumor bearing mice were used for the experiment.
(2) Observation of imaging of small peptide probe in nude mice cancer models. Each tumor-bearing mouse (NCl-H1975, L78, MCF7, HT-29 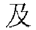 HepG2) was injected with 0.1 ml (2.96MBq) of small peptide probe ^{99m}Tc-cNGEGQQc via a tail vein respectively and scanned by SPECT. ^{99m}Tc labeled non-related small peptides cNAQAEQ_{C} was used as a negative control for small peptide probe. To monitor the distribution of molecular probe in vivo, SPECT images were acquired immediately and at 0.5h, 1 h, 2h, 3h, 5h, 6h and 24h after injection of small peptide probe. Imaging at first time point and peak uptake time was also recorded. All these data were used to evaluate the imaging features of the probe in two different lung cancer cells and other cancer cells.

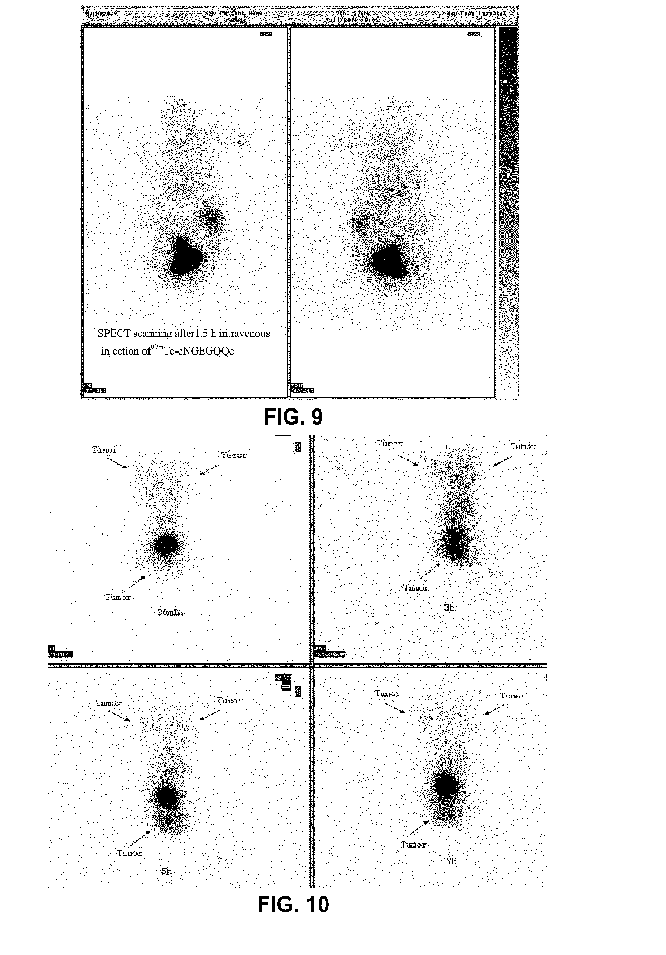

SPECT imaging of animals injected with small peptide probe demonstrated major distribution to the kidneys and bladder and, to a lesser extent, to the liver. Low radioactivity was also observed in the intestine, limbs, head and chest. The increased intense radiotracer activity in bladder was shown while decreased uptake in the tumor (L78) (Figure 10).

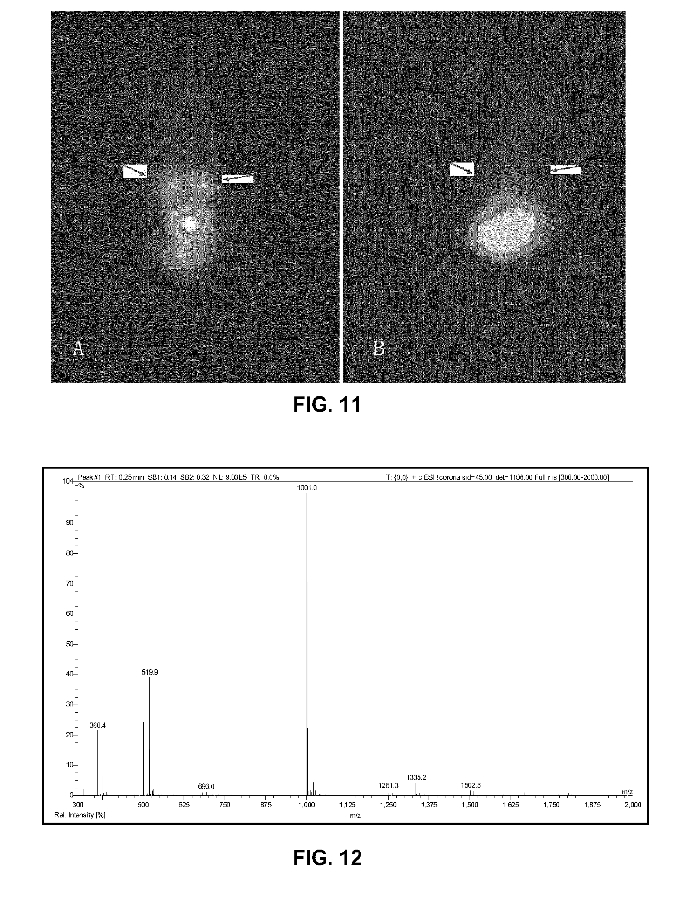

Tumor imaging was vaguely at 30 min after the injection of small peptide probe. With the tumor uptake gradually increasing, the image became clear visualization. Figure 11 showed that the tumor (H1975) was clearly visualized at 2h after injection of small peptide probe, while the tumor image was blurry after injection of the control probe.

(3) Biodistribution of small peptide probe ^{99m}Tc-cNGEGQQc in nude mice cancer models

The maximum tumor-specific accumulation occurred at 2h after injection of molecular probe, while the highest kidney uptake and lowest brain uptake were observed postinjection. The target/non-target (T/NT) ratios were presented that small peptide probe had the higher tumor-to-brain (10.32) and tumor-to-muscle (4.76) ratios and lower tumor-to-blood ratio (1.46) (Table 4).

**Table 4 T/NT ratio of ^{99m}Tc-cNGEGQQc in mice bearing lung cancer (n = 3)**

| Tumor/organ | T/NT ratio |
|---|---|
| Tumor/liver | 0.47±0.13 |
| Tumor/brain | 10.32±4.26 |
| Tumor/kidney | 0.23±0.18 |
| Tumor/lung | 0.92±0.17 |
| Tumor/heart | 4.43±0.75 |
| Tumor/bone | 1.82±0.84 |
| Tumor/muscle | 4.76±0.79 |
| Tumor/stomach | 1.19±0.11 |
| Tumor/small intestine | 1.11±0.32 |
| Tumor/blood | 1.46±0.26 |

As stated above, the tumor lesions (adenocarcinoma and squamous carcinoma of lung cancer) were clearly visualized after injection of small peptide probe. Meanwhile, no visual imaging was shown after injection of control probe. Similar results were not achieved in breast cancer, colon cancer and hepatocellular carcinoma models after injection of small peptide probe. These data suggest that the peptide of the invention can be used as a carrier for delivery of diagnostic radioisotope ^{99m}Tc to lung cancer zenografts through the blood circulation, and specifically bind to lung cancer cells.

Peptide of this invention may be used in combination with known imaging agents. The imaging agents comprise ^{99m}Tc, ¹¹¹In, ¹⁸F-FDG, ⁶⁸Ga, ⁶⁴Cu.

### Example 2

### 1 Preparation of radiotherapeutic agent (¹³¹I-cNGEGQQc) for lung cancer

According to the procedure in example 1, cNGEGQQc, tyrosine and condensing agent EDC-HCl (1-ethyl-(3-dimethylaminopropyl) carbodiimide hydrochloride amine) was mixed at a molar ratio of 1:3:3.6. The reaction time was 2 hours at room temperature. The small peptide cNGEGQQc-Tyr complex was synthesized by condensation reaction between the N-terminal of peptide and C-terminal of tyrosine (Figure 12).

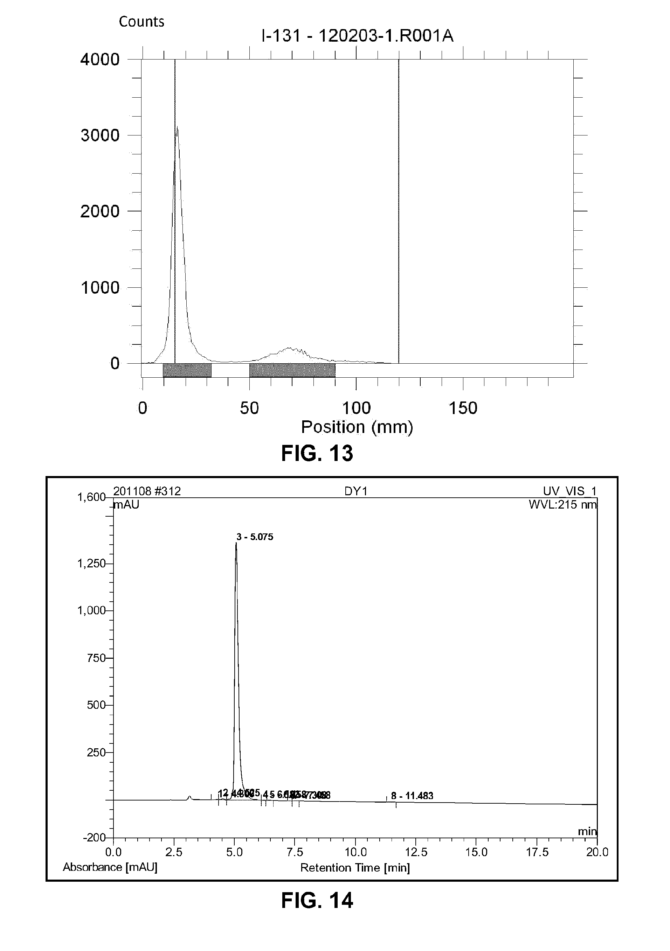

For labeling the small peptide cNGEGQQc with iodine-131 using the chloramine-T method, the procedure was performed according to the following manner:
Peptide-Tyr complex stated above (50 µg) was completely dissolved in 50µl of PBS buffer (0.5M, pH=7.4), then was added to 20 µl of ¹³¹I (37MBq), followed by 30µl of chloramine-T (3µg/µl) (final concentration 0.9µg/µl). The component was mixed in a shaker for 2 min and the reaction was terminated by adding 45µl of sodium thiosulfate (4µg/µl). The reaction mixture was finally added 200µl of PBS buffer (0.5M, pH=7.4). The peptides were determined by paper chromatography. A drop of the labeled peptides were placed on the paper, then dipped into a mixture liquid with n-butanol: ethanol: ammonia (5: 1: 2). Labeling efficiency of the labeled peptides was measured using radioactive thin-layer scanner. The labeled peptides were purified using gel filtration on Sephadex G-25 column. The radiochemical purity of purified peptides was measured using paper chromatography (Figure 13).

The preparation and purification of labeled peptide by Sephadex G25 were as follow: 1 g of Sephadex (dextran gel) 25 were soaked in PBS (pH=7.4) for 24 h. The fine particles were removed by gently shaking. After the Sephadex G25 was completely hydrated, pumping decompression was used to remove the air bubbles. Sephadex G25 was then added into a glass chromatography tube. PBS (pH=7.4) and BSA (20 mg dissolved in I mL of PBS) were added into the tube separately. After washing with PBS (pH=7.4), the reaction solution was filtered through the column. The eluate was monitored by absorbance at 280 nm and added appropriate amount of BSA and NaN₃, followed by lyophilizing and aliquoting for future use.

### 2 Analysis of labeling efficiency and stability of small molecular targeted radiotherapeutic agent (¹³¹I-cNGEGQQc) for lung cancer in vitro

Labeling efficiency of radiotherapeutic agent was determined by paper chromatography. The details were as follow: A drop of the radiotherapeutic agent was placed on one side of the paper, then dipped into a mixture liquid with n-butanol: ethanol: ammonia (5: 1: 2). The separation occurs as the liquid moves along the paper. Take the paper out and dry it when the liquid moves to the other side of paper. Paper was cut into ten equal pieces and put into the tube separately. The radioactivity of each pieces of paper was measured by γ radioactivity counter and calculated labeling rate (radioactivity peaks of unpurified labeled peptide / sum of each radioactivity peakX100%) and radiochemical purity (radioactivity peaks of purified labeled peptide / sum of each radioactivity peakX100%). Rf of ¹³¹I-labeled peptide was 0-0.1 and Rf of free¹³¹I was 0.4-0.6 and 0.9-1.0. The optimal conditions for labeling cNGEGQQc with ¹³¹I were as follows: The best peptides/chloramine-T weight ratio was 1:1.8. Reaction conditions were at pH 7.4 and 20°C for 2min. The labeling rate of peptides using ¹³¹I in the optimal conditions was over 90%. Figure 14 present HPLC radiochromatograms of ¹³¹1-labeled peptide.

To evaluate the stability of radiotherapeutic agent at room temperature and in fresh human serum for 24h, radiochemical purity was measured using paper chromatography. After purification with HPLC, the radiochemical purity of ¹³¹1-labeled cNGEGQQc was> 90% at room temperature for 24h. To further estimate the stability of small peptide probe in simulation environment in vivo, the probe was placed in fresh human serum at 37°C for 24h. After 24 h incubation, the radiochemical purity was 92.5% at 0 h and 88.2% at 24h respectively. These data suggests that the small molecular radiotherapeutic agent is very stable in serum

### 3 Analysis of biodistribution of small molecular targeted radiotherapeutic agent for lung cancer in vivo

(1) Fifteen normal Kunming male mice (4-6 weeks old, weight 19-21 g) were injected with 50µl (0.48MBq) of ¹³¹1-labeled cNGEGQQc via a tail vein respectively. At 1, 3, 6, 12, and 24h, three animals were anesthetized and sacrificed by cervical dislocation. Whole blood was collected and organs of interest were removed and weighed. The amount of radioactivity in blood and each organ was determined and calculate the percentage of the injected dose per gram of tissue (%ID/g) after radioactive decay correction. SPECT (Millennium VG; GE Healthcare) was equipped with a low-energy, high resolution collimator. Images were acquired using energy peak centered at 364KeV, an energy window of 20% and a 128X128 matrix at a magnification of 1.0.

SPECT imaging showed that the kidney had the highest radioactivity levels and longer clearance among all organs in healthy mice, indicating predominant renal excretion of ¹³¹1-labeled cNGEGQQc. During the observation period, the radioactivity level in various organs gradually decreased, while the radioactivity of gastrointestinal was relatively stable. It indicated that the stability of ¹³¹1-labeled cNGEGQQc was excellent and no free ¹³¹1 release in vivo. The least uptake of ¹³¹1-labeled cNGEGQQc was observed in muscles and brain. The biodistribution ¹³¹1-labeled cNGEGQQc in mice was summary in Table 5.

**Table 5 Biodistribution ¹³¹1-labeled cNGEGQQc in mice (%ID/g)**

| Tissues | 1 h | 3h | 6h | 12h | 24 h |
|---|---|---|---|---|---|
| liver | 0.3830±0.0018 | 0.4152±0.0024 | 0.1403±0.0004 | 0.0829±0.0007 | 0.0338±0.0001 |
| spleen | 0.2034±0.0008 | 0.3137±0.0023 | 0.1841±0.0002 | 0.0770±0.0005 | 0.0384±0.0001 |
| kidney | 2.4804±0.0059 | 2.0763±0.0072 | 0.9813±0.0025 | 0.3812±0.0023 | 0.2028±0.0006 |
| lung | 0.3773±0.0012 | 0.3896±0.0021 | 0.2395±0.0008 | 0.2879±0.0023 | 0.0765±0.0001 |
| stomach | 0.6943±0.0034 | 1.2331±0.0095 | 0.8734±0.0035 | 0.7219±0.0105 | 0.0696±0.0005 |
| intestine | 0.2910±0.0011 | 0.3185±0.0012 | 0.1956±0.0004 | 0.1354±0.0009 | 0.0723±0.0003 |
| muscle | 0.0800±0.0002 | 0.0874±0.0005 | 0.0515±0.0004 | 0.0394±0.0003 | 0.0206±0.0001 |
| bone | 0.2420±0.0005 | 0.2517±0.0009 | 0.1604±0.0006 | 0.0887±0.0004 | 0.0702±0.0001 |
| brain | 0.0279±0.0007 | 0.0273±0.0002 | 0.0159±0.0004 | 0.0183±0.0001 | 0.0170±0.0001 |
| blood | 0.4297±0.0019 | 0.5124±0.0028 | 0.3971 ±0.0016 | 0.1788±0.0017 | 0.0644±0.0002 |

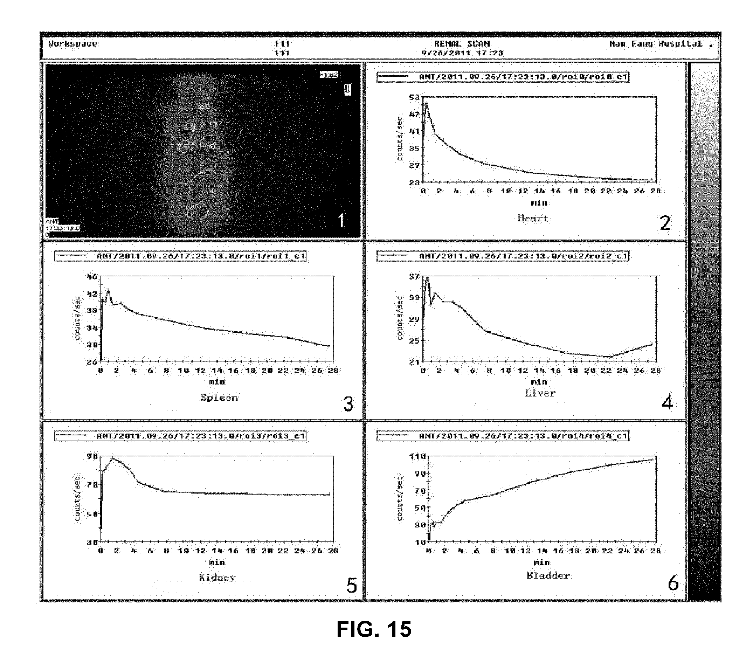

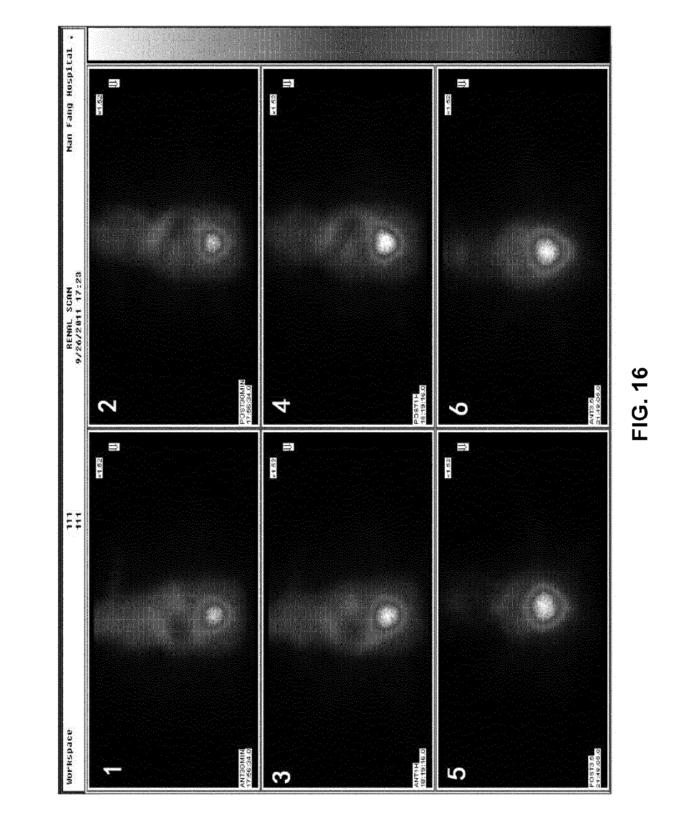

(2) Two of healthy Japanese male white rabbits were fixed in supine position on a wooden experimental stage. SPECT collimator was set on the rabbit thoracic and abdominal to ensure that the whole body of rabbit was within vision field of SPECT imaging. The injectate of ¹³¹1-labeled cNGEGQQc diluted with saline water (0.5 ml/14.8 MBq/per rabbit) was administered through the ear vein injection. The images were obtained in two ways in order to evaluate and compare dynamic imaging with multitemporal static imaging. First, the images were acquired immediately at a rate of 1 frame/10sec x6 after injection of the ¹³¹1-cNGEGQQc, followed by a rate of 1 frame/1 min x4 and 1 frame/5 min x5. Second, multitemporal static images of anterior and posterior were obtained following the dynamic imaging at 0.5h, 1h and 3.5h after injection. The posterior dynamic images were analyzed by ROI semi-quantitatively. The time-radioactivity curves of main organs including precordia, liver, spleen, kidney and bladder were obtained respectively by ROI analysis (Figure15-16).

### 4 Inhibition of lung cancer growth by small molecular targeted radiotherapeutic agent for lung cancer

Animal models of human tumors. Two of human non-small cell lung cancer cell lines including NCl-H1975(adenocarcinoma)and L78 (squmous carcinoma) were used in the study. These cells were cultured in conventional methods. When cells grew at logarithmic phase, cells were then harvested with trypsin and centrifuged to remove trypsin digestion solution. After washing with PBS twice, the cells were re-suspended in free serum culture medium at a concentration of 5X10⁶ cells / ml. The suspended cells (0.2 ml) were inoculated s.c. into the back of nude mice to establish the lung cancer models, respectively. There are twelve nude mice in each group of cancer model. Tumor growth and general states such as mental, diet and weight were monitored periodically. When tumors reached approximately 1 cm in mean diameter, the tumor bearing mice were used in therapeutic efficacy studies.

All mice received a solution of 0.2% potassium iodine orally to block uptake of free iodine-131 by the thyroid beginning from 3 days before treatment and to end of experiment. Twelve tumor bearing mice of each lung cancer cell line were randomly divided into four groups with three animals each and injected with ¹³¹I-cNGEGQQc, ¹³¹I-cNAQAEQc (negative peptide control), ¹³¹I and normal saline via a tail vein, respectively. The tumor size was measured two dimensionally on days 3, 6, 9, 12, 15, 18, 21, 24, 27 and 30 after injection, while mice weight was also determined. The tumor volume was calculated by the formula volume V = (4/3) x π x R1 x R2, where R1 is radius 1 and R2 is radius 2 and R1 < R2.

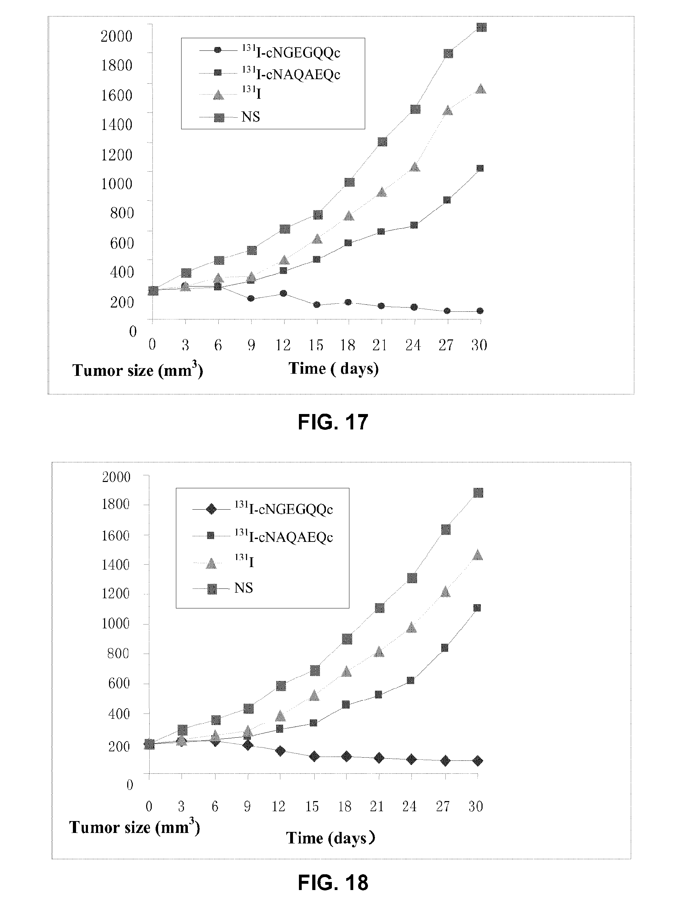

Growth curves of the tumors were constructed according to these tumor volumes. The tumor size of H1975 and L78 in ¹³¹I-cNGEGQQc treated groups decreased on days 7 after injection, while tumor grew continentally in the control groups (Figure 17-18). The median survival time of each group was as follows: 54 days in mice with ¹³¹I-cNGEGQQc; 45 days in mice with ¹³¹I-cNAQAEQc; 42 days in mice with ¹³¹I and 43 days in mice with normal saline. These results suggested that radiotherapeutic agent of the invention can significantly inhibit lung cancer growth in vivo.

### 5 Evaluation of toxicity of small molecular targeted radiotherapeutic agent to major organs

### (1) Analysis of toxicity of radiotherapeutic agent in mice bearing lung cancer cells

After treatment with radiotherapeutic agent for three weeks, the mice were sacrificed and the major tissues or organs such as blood, liver, kidneys, heart, lungs and spleen were removed completely. Blood cell and liver and kidney function were analyzed by the routine test. Regular pathological morphology of preserved organs and tissues such as liver and kidney were examined using microscopy. Ultrastructural observations were performed on the preserved organs and tissues stated above using electromicroscopy.

The hematological analysis showed no significant changes of RBC, WBC and PLT in the ¹³¹I-cNGEGQQc treatment groups as compared to the normal saline groups. The leukocyte count showed decrease between the control and ¹³¹I-cNAQAEQc or ¹³¹I groups. The data from the serum biochemical examinations demonstrated that there were no statistically significant differences of liver function (AST and ALT) and kidney function (CRE and BUN) in either the control or treated group(P<0.05)(Table 6). No histopathological and ultrastructural changes were observed in the organs including liver, kidneys, heart and lungs.

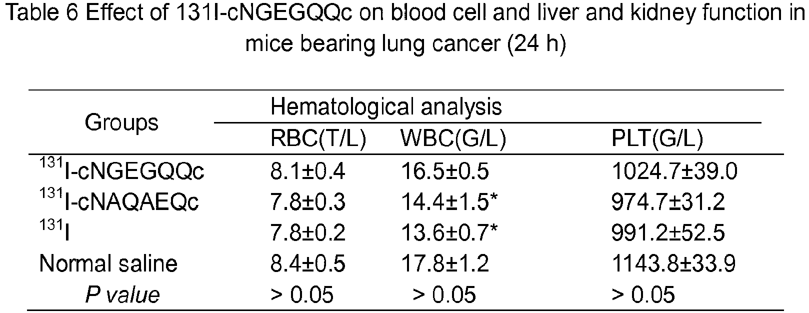

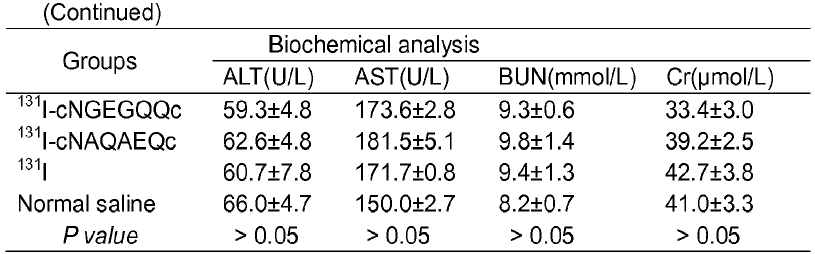

### (2) Analysis of toxicity of radiotherapeutic agent in normal rabbits

Six healthy rabbit were randomly divided into two groups with three animals each and injected with ¹³¹I-cNGEGQQc and normal saline via a tail vein, respectively. Body temperature of each rabbit was measured at 15 min before injection and at 1 h, 12h and 24 h after injection. The temperature range of each rabbit at different time point was as follow: 38.9-3 9.3°C before injection; 38.7-39.2°C at 1 h after injection; 39.1-39.6°C at 12 h after injection; 38.9-39.1°C at 24 h after injection. The largest temperature variance of each animal was below 0.7°C during 24 h and below 1.5°C in three tests. The blood samples were drawn from the ear vein of the rabbits for testing the count of blood cell (including RBC, WBC and PLT) and liver function (AST and ALT) and kidney function (CRE and BUN).The hematological analysis, liver and kidney function at three time-points were listed in Table 7. Based on the analysis results of the above-mentioned parameters, no significant differences were found between the treatment and the control group (P<0.05), except for a decrease of PLT at 24 h after treatment. No significant differences were also found in respiratory, autonomic and central nervous system and behavior pattern. These observations clearly suggest there is no toxicity of ¹³¹I-cNGEGQQc to major organs including lungs, liver, kidneys and heart.

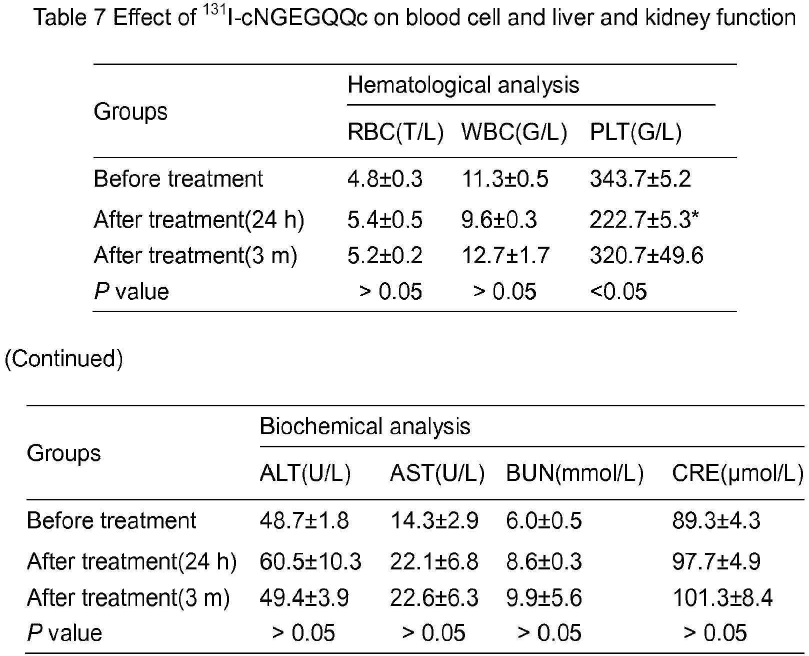

In this example, the radiotherapeutic isotope ¹³¹I is used for preparing a radiotherapeutic agent for a non-small cell lung cancer. Optionally, ¹⁸⁸Re and ⁹⁰Y is also are good choices.

The examples described above are preferred embodiments of the invention. For the skilled person in the field, any apparent changes in the invention without departing from the spirit and scope for improvement should be considered part of the invention.

## Claims

1. A small peptide, comprising 8 amino acids with a sequence of XGXG, wherein: "G" represents L- Glycine (Gly), "X" represents any one of 20 amino acids.

2. The small peptide of claim 1, **characterize in that** the amino acid sequence of the peptide is cNGEGQQc (SEQ ID NO.1), where "c" represents d-cysteine (Cys), "N" represents L-Asparagine (Asn), "G" represents L-Glycine (Gly), "E" represents L-Glutamic acid (Glu), "Q" represents L-Glutamine (Gln).

3. A small peptide probe being obtained by labeling a peptide with a radiotherapeutic isotope; the peptide comprising 8 amino acids with the sequence of XGXG, wherein: "G" represents L- Glycine (Gly), "X" represents any one of 20 amino acids.

4. The small peptide probe of claim 3, **characterize in that** the amino acid sequence of the peptide is cNGEGQQc (SEQ ID NO.1), where "c" represents d-cysteine (Cys), "N" represents L-Asparagine (Asn), "G" represents L-Glycine (Gly), "E" represents L-Glutamic acid (Glu), "Q" represents L-Glutamine (Gln).

5. The small peptide probe of claim 3, **characterize in that** the radiotherapeutic isotope is ^{99m}Tc, ¹¹¹In, ¹⁸F-FDG, ⁶⁸Ga, or ⁶⁴Cu.

6. A method for producing a small peptide probe, the method comprising:
(1) conjugating a peptide to a chelator of S-acetyl-mercaptoacetyltriglycine (MAG₃) to produce a small peptide-MAG₃ complex; and
(2) labeling the peptide-MAG₃ complex by a radiotherapeutic isotope to generate the small peptide probe;
wherein the peptide comprises 8 amino acids with the sequence of XGXG, where: "G" represents L- Glycine (Gly), "X" represents any one of 20 amino acids, and the radiotherapeutic isotope is ^{99m}Tc.

7. The method of claim 6, **characterized in that** the peptide comprises 8 amino acids with the sequence of cNGEGQQc (SEQ ID NO.1), wherein: "c" represents d-cysteine (Cys), "N" represents L-Asparagine (Asn), "G" represents L-Glycine (Gly), "E" represents L-Glutamic acid (Glu), "Q" represents L-Glutamine (Gln).

8. Use of a small peptide probe of any one of claims 3-5 for producing a molecular imaging diagnostic agent of lung cancer.

9. A small molecular radiotherapeutic agent for lung cancer, the agent being obtained by labeling a peptide with a radiotherapeutic isotope, the peptide comprising 8 amino acids with the sequence of XGXG, where: "G" represents L- Glycine (Gly), "X" represents any one of 20 amino acids.

10. The small molecular radiotherapeutic agent of claim 9, **characterized in that** the peptide comprises 8 amino acids with the sequence of cNGEGQQc (SEQ ID NO.1), wherein: "c" represents d-cysteine (Cys), "N" represents L-Asparagine (Asn), "G" represents L-Glycine (Gly), "E" represents L-Glutamic acid (Glu), "Q" represents L-Glutamine (Gln).

11. The small molecular radiotherapeutic agent of claim 9, **characterized in that** radiotherapeutic isotope is ¹³¹I, ¹⁸⁸Re, or ⁹⁰Y.

12. A method for producing a small molecular targeted radiotherapeutic agent for lung cancer, the method comprising:
1) coupling a N-terminal of a peptide to tyrosine; and
2) dissolving the peptide coupling with tyrosine on its N-terminal, adding a radiotherapeutic isotope and chloramine-T respectively, mixing a resulting mixture in a shaker for 2 min, and adding sodium thiosulfate for stopping the reaction;
wherein the peptide comprises 8 amino acids with the sequence of XGXG, where: "G" represents L- Glycine (Gly), "X" represents any one of 20 amino acids, and the radiotherapeutic isotope is ¹³¹I.

13. The method of claim 12, **characterized in that** the peptide comprises 8 amino acids with the sequence of cNGEGQQc (SEQ ID NO.1), wherein: "c" represents d-cysteine (Cys), "N" represents L-Asparagine (Asn), "G" represents L-Glycine (Gly), "E" represents L-Glutamic acid (Glu), "Q" represents L-Glutamine (Gln).

14. The method of claim 12, **characterized in that** step (1) is detailed as follows: mixing the small peptide cNGEGQQc and tyrosine, adding a condensing agent EDC-HCl (1-ethyl-(3-dimethylaminopropyl) carbodiimide hydrochloride amine), allowing a resulting mixture to reaction at room temperature for 2 hours so that the N-terminal of the peptide is coupled to C-terminal of tyrosine by condensation reaction.

15. The method of claim 12, **characterized in that step** (2) is detailed as follows: diluting peptide-Tyr complex in PBS buffer (0.5 M, pH=7.4); after the peptide is completely dissolved in the buffer, adding ¹³¹I (37 MBq/20 µl) and chloramine-T (final concentration 0.9 µg/µl); mixing a resulting mixture in a shaker for 2 min and then adding 45 µl of sodium thiosulfate (4 µg/µl) for stopping the reaction.


SEQUENCE LISTING

<110>  Linlang, Guo
       Guiping, Li
       Zhenzhu, Chen
       Yajie, Liu

<120>  SMALL MOLECULAR PEPTIDE AND USE THEREOF

<130>  GZNK-00102-NEP

<160>  1

<170>  PatentIn version 3.5

<210>  1
<211>  8
<212>  PRT
<213>  Artificial Sequence

<220>
<223>  Fully synthetic peptide

<400>  1

Cys Asn Gly Glu Gly Gln Gln Cys
1               5